Europäisches Patentamt

European Patent Office

Office européen des brevets

(11) Veröffentlichungsnummer: **0 413 246 A2**

# EUROPÄISCHE PATENTANMELDUNG

(21) Anmeldenummer: 90115215.7

(22) Anmeldetag: 08.08.90

(51) Int. Cl.5: **A61K 9/00**, A61K 9/08, A61K 31/505

(30) Priorität: 18.08.89 CH 3015/89

(43) Veröffentlichungstag der Anmeldung:
20.02.91 Patentblatt 91/08

(84) Benannte Vertragsstaaten:
AT BE CH DE DK FR GB IT LI LU NL SE

(71) Anmelder: F.HOFFMANN-LA ROCHE & CO.
AKTIENGESELLSCHAFT
Postfach 3255
CH-4002 Basel(CH)

(72) Erfinder: Ferro, Alberto, Dr.
Gstaltenrainweg 67
CH-4125 Riehen(CH)
Erfinder: Wyss, Pierre-Charles, Dr.
Unterwartweg 27
CH-4132 Muttenz(CH)

(74) Vertreter: Grossner, Lutz, Dr. et al
Grenzacher Strasse 124 Postfach 3255
CH-4002 Basel(CH)

(54) Verfahren zur Herstellung parenteraler Applikationsformen, Kit zum Gebrauch dazu, und das neue 5-Fluorcytosin-monohydrochlorid.

(57) Parenteral verabreichbare Lösungen aus stark sauren oder stark basischen Salzen pharmazeutischer Wirkstoffe werden durch Vereinigen eines solchen Salzes mit einem neutralisierenden Solvens hergestellt.

EP 0 413 246 A2

# VERFAHREN ZUR HERSTELLUNG PARENTERALER APPLIKATIONSFORMEN, KIT ZUM GEBRAUCH DAZU, UND DAS NEUE 5-FLUORCYTOSIN-MONOHYDROCHLORID

Die Bereitstellung von parenteral verabreichbaren Lösungen pharmazeutischer Wirkstoffe ist häufig mit technischen Schwierigkeiten verbunden. Solche Schwierigkeiten treten zum Beispiel dann auf, wenn die hergestellten Lösungen nicht hinreichend stabil sind, um eine längere Lagerung, gegebenenfalls unterhalb (Ausfällung der Wirksubstanz) oder oberhalb normaler Raumtemperaturen (Zersetzung des Wirkstoffs), zu gewährleisten. Derartige Schwierigkeiten versucht man dadurch zu umgehen, dass die Lösungen in situ, d.h. unmittelbar vor Gebrauch hergestellt werden. Die Auflösungsgeschwindigkeit des Wirkstoffs ist aber oft unbefriedigend, insbesondere dann, wenn die Konzentration des Wirkstoffs im gewünschten Lösungsmittelvolumen nahe der Sättigungsgrenze ist. Das Problem wird noch grösser, wenn der zu lösende Wirkstoff nicht lyophilisierbar ist und als Sterilpulver vorliegen muss. Ein Ausweg kann im Falle ionisierbarer Wirkstoffe gefunden werden, indem ein genügend lösliches und im trockenen Zustand stabiles Salz hergestellt wird. Salze sehr schwacher Basen oder sehr schwacher Säuren mit starken Säuren resp. starken Basen erweben jedoch derart saure (pH <3) resp. stark basische Lösungen (pH >9,5), dass deren parenterale Anwendung nicht oder nur nach Neutralisation zu physiologischen pH-Werten möglich ist. So ist zur Herstellung von parenteralen Lösungen von Metronidazol, dessen Hydrochlorid sich in Wasser mit stark saurer Reaktion löst, ein Präparat im Handel, bei dem die Herstellung der gebrauchsfertigen Lösung zwei Operationen erfordert. Zunächst wird das Hydrochlorid in Wasser gelöst, worauf in einer zweiten Operation die erhaltene wässrige Lösung auf einen physiologischen pH-Wert neutralisiert wird. Dieses Verfahren ist nicht nur sehr umständlich und kontaminationsanfällig, sondern birgt auch die Gefahr in sich, dass die Neutralisation der stark sauren Lösung versehentlich unterbleibt.

Es wurde nun überraschend gefunden, dass die Herstellung von physiologisch verträglichen parenteral verabreichbaren Lösungen aus derart stark sauren resp. stark basischen Salzen pharmazeutischer Wirkstoffe direkt, d.h. nur in einem einzigen Schritt verwirklicht werden kann. Erfindungsgemäss wird ein Verfahren zur direkten Herstellung physiologisch verträglicher parenteral verabreichbaren Lösungen aus stark sauren resp. stark basischen Salzen pharmazeutischer Wirkstoffe bereitgestellt, welches dadurch gekennzeichnet ist, dass man ein hinreichend lösliches Salz derartiger Wirkstoffe mit einem ausreichenden Volumen eines neutralisierenden Solvens zusammenbringt und gegebenenfalls entstehende und vorübergehend ausfallende Wirkstoff-Base resp. Wirkstoff-Säure durch leichtes Schütteln in Lösung bringt.

Der hier verwendete Ausdruck "stark saure resp. stark basische Salze pharmazeutischer Wirkstoffe" soll Salze von pharmazeutischen Wirkstoffen bezeichnen, die ein pKs von < 4 (sehr schwache Basen) resp. > 8-9 (sehr schwache Säuren) aufweisen. Die Lösungen dieser Salze weisen pH-Werte von < 2,5 resp. > 9,5 auf, die vom physiologisch akzeptablen pH-Bereich von 4 bis 8 weit entfernt sind. Beispiele sehr schwacher Basen sind 5-Fluorcytosin und Metronidazol. Weitere Beispiele sind Fluorazapam, Levodopa, Flunitrazepam, Bromazepam. u.s.w. Beispiele sehr schwach saurer Wirkstoffe sind 5-Fluorouracil, Allobarbital, Acetaminophen, Butabarbital, Hexobarbital, Paracetamol, Pentobarbital, Phenobarbital, Phenytoin u.s.w.

Als Basen für die Neutralisation stark saurer Lösungen derartiger Salze kommen alle pharmazeutisch anwendbaren Basen in Betracht. Vorzugsweise verwendet man Basen, mit denen bei der Neutralisation auch eine optimale iso-osmotisierende Wirkung erzielt wird, wie z.B. Dinatriummonohydrogenphosphat, Tris(hydroxymethyl)-aminomethan, L-Arginin oder L-Lysin, vorzugsweise im Gemisch mit Natriumhydrogencarbonat, das beim Neutralisationsvorgang $CO_2$ freigibt und dadurch den Prozess der Auflösung der gegebenenfalls entstehenden und ausfallenden Wirkstoffbase bzw. -säure wesentlich beschleunigt.

Als Säuren für die Neutralisation stark basischer Lösungen derartiger Wirkstoffsalze können alle pharmazeutisch anwendbaren Säuren in Betracht gezogen werden. Vorzugsweise verwendet man Säuren, mit denen bei der Neutralisation eine optimale iso-osmotisierende Wirkung erzielt wird, wie z.B. Citronensäure, Essigsäure, Mononatriumdihydrogenphosphat, Milchsäure u.s.w. Das zur Beschleunigung des Auflösungsprozesses vorzugsweise zu verwendende Natriumhydrogencarbonat kann in diesem Falle dem Wirkstoffsalz beigemischt werden.

Die erfindungsgemäss erhältlichen Lösungen können weiterhin übliche pharmazeutische Hilfsstoffe, wie Mittel zur Isoosmotisierung oder pH-Einstellung, sowie Puffer und/oder Stabilisatoren enthalten. Diese Hilfsstoffe können dem Wirkstoffsalz zugemischt werden oder können im Solvens gelöst vorliegen.

In einer bevorzugten Ausführungsform betrifft die Erfindung die Herstellung von parenteralen Lösungen von 5-Fluorcytosin, wobei als Salz das 5-Fluorcytosin-Monohydrochlorid verwendet wird.

5-Fluorcytosin-Monohydrochlorid wurde bisher nirgends beschrieben und ist ebenfalls Gegenstand der Erfindung. Die Verbindung kann wie weiter unten beschrieben, durch Zusatz von Aethanol zu einer wässrigen Lösung des in situ mit Salzsäure hergestellten 5-Fluorcytosin-Salzes gewonnen werden.

Die erfindungsgemäss in einem einzigen Schritt herstellbaren parenteralen Lösungen werden zweckmäsig unmittelbar vor dem Gebrauch hergestellt. Die Erfindung betrifft daher auch ein Kit, d.h. ein aus Einzelteilen bestehendes Erzeugnis, umfassend:

a) einen Behälter, in dem sich ein stark saures resp. stark basisches Salz eines pharmazeutischen Wirkstoffes befindet, das hinreichend löslich und im trockenen Zustand stabil ist;

b) einen Behälter, in dem sich ein ausreichendes Volumen eines neutralisierenden Solvens befindet, das die entstehende Wirkstoff-Base resp. Wirkstoff-Säure auflösen kann; und

c) ein Verbindungsstück, das für die Ueberführung des Wirkstoffsalzes in das neutralisierende Solvens geeignet ist.

Für eine solche Vorrichtung kann z.B. das unter dem Namen "ADD-Vantage" (Abbot Laboratories, North Chicago, III.USA) im Handel befindliche Infusionssystem verwendet werden.

Die Erfindung wird durch die nachstehenden Beispiele weiter erläutert.

### Beispiel 1

| A. Wirkstoff | |
| --- | --- |
| 5-Fluorcytosin Monohydrochlorid | 3,21 g |

Der Wirkstoff wird in ein Glasfläschchen à 20 ml eingebracht, das mit einem Gummistopfen verschlossen wird.

| B. Solvens | |
| --- | --- |
| Natriumhydrogencarbonat | 1,375 g |
| Dinatriummonohydrogenphosphat. Dodecahydrat | 3,275 g |
| HCl 1N ad pH 8,0 | q.s. |
| Wasser zu Injektionszwecken q.s. ad | 250,0 ml |

Das Solvens wird unter Vakuum in einen Infusionsbeutel à 500 ml, z.B. einen Infusionsbeutel des ADD-Vantage-Infusionssystems, abgefüllt.

Zur Herstellung der gebrauchsfertigen Infusionslösung wird das Wirkstoffsalz in das neutralisierende Solvens überführt, worin die darin entstehende 5-Fluorcytosin-Base durch leichtes Schütteln in weniger als 1 Minute klar gelöst werden kann. Die erhaltene Losung enthält 2,5 g 5-Fluorcytosin in 250 ml und weist einen pH-Wert von ca. 5,9-6,0 auf. Die nicht neutralisierte Lösung von 5-Fluorcytosin-Monohydrochlorid würde einen pH-Wert von ca. 1,5 aufweisen.

Die vollständige Auflösung mikronisierter 5-Fluorcytosin-Base in 250 ml einer handelsüblichen Infusionslösung würde eine weit mehr als 8 Minuten kräftiges Schütteln benötigen, was offensichtlich für eine praktische Anwendung nicht in Frage kommt.

### Beispiel 2

| A. Wirkstoff | |
|---|---|
| Misonidazol-Hydrochlorid | 590,6 mg (entspr. 500 mg Misonidazol) |
| B. Solvens | |
| Natriumhydrogencarbonat | 0,55 g |
| Dinatriummonohydrogenphosphat-Dodecahydrat | 1,31 g |
| HCl 1N ad pH 7,5 | q.s. |
| Wasser zu Injektionszwecken q.s. ad | 100,0 ml |

Das Solvens wird unter Vakuum in einen handelsüblichen PVC-Infusionsbeutel à 250 ml abgefüllt.

Zur Herstellung der gebrauchsfertigen Infusionslösung wird zunächst das Misonidazol-Hydrochlorid mit Hilfe eines geeigneten Verbindungsstückes (z.B. einer sogenannten Transfernadel) in einem Teil des Solvens suspendiert. Die erhaltene Suspension wird dann in den Infusionsbeutel eingezogen, wo sich schliesslich das entstehende Misonidazol auflöst. Der pH-Wert der Lösung, welche 100 mg Misonidazol in 100 ml enthält, beträgt ca. 6,9. Die nicht neutralisierte Lösung wurde einen pH-Wert von < 2 aufweisen.

Beispiel 3

| A. Wirkstoff | |
|---|---|
| Flurazepam-Dihydrochlorid | 17,82 mg (entsprechend 15 mg Flurazepam) |

Das Wirkstoffsalz wird in ein Glasfläschchen à 10 ml eingebracht, das mit einem Gummistopfen verschlossen wird.

| B. Solvens | |
|---|---|
| L-Arginin | 6,5 mg |
| Natriumchlorid | 85,0 mg |
| Wasser zu Injektionszwecken q.s. ad | 10,0 ml |

Das Solvens wird in ein sterilisiertes PE-Fläschchen à 10 ml eingefüllt.

Zur Herstellung der gebrauchsfertigen Injektionslösung wird das partiell neutralisierende Solvens mit Hilfe einer Transfernadel in das Wirkstofffläschchen überführt, wo das Flurazepam durch leichtes Schütteln in Form des entstehenden Monohydrochlorides aufgelöst wird. Der pH-Wert der erhaltenen Lösung, welche 15 mg Flurazepam in Form des Monohydrochlorides in 10 ml enthält, beträgt 7,4. Die nicht neutralisierte Lösung des entsprechenden Dihydrochlorids würde einen pH-Wert von ca. 2,4 aufweisen.

Beispiel 4

| A. Wirkstoff | |
|---|---|
| 5-Fluoruracil | 250,0 mg |
| NaOH 1N | 1,923 ml |
| Wasser zu Injektionszwecken q.s. ad | 5,0 ml |

Die Lösung wird in ein Glasfläschchen à 20 ml abgefüllt und wie üblich lyophilisiert.

| B. Solvens | |
| --- | --- |
| Citronensäure-Monohydrat | 40,0 mg |
| Wasser zu Injektionszwecken q.s. ad | 5,0ml |

Das Solvens wird in ein sterilisiertes PE-Fläschchen à 10 ml eingefüllt.

Zur Herstellung der gebrauchsfertigen Injektionslösung wird das Solvens mit Hilfe einer Transfernadel ins Wirkstofffläschchen überführt, wo sich das zum Teil entstehende freie 5-Fluorouracil durch leichtes Schütteln auflöst.

Der pH-Wert der erhaltenen Lösung, welche 250 mg 5-Fluorouracil (nur z.T. in Form des Natriumalzes) enthält, beträgt 8,4. Die nicht neutralisierte Lösung würde einen pH-Wert von mehr als 10 aufweisen.


## Beispiel 5


Das 5-Fluorcytosin-Monohydrochlorid kann wie folgt hergestellt werden:

200 g 5-Fluorcytosin werden in 720 ml 25%-iger Salzsäure bei 70° C gelöst. Anschliessend werden zu der Lösung unter Kühlen im Eisbad und Rühren 8 l Aethanol zugetropft. Danach wird das kristallisierende Gemisch eine Stunde bei 5° C weiter gerührt. Die Kristalle werden abgenutscht, mit Aethanol gewaschen und bei 80° C getrocknet.


## Ansprüche

1. Verfahren zur Herstellung physiologisch verträglicher parenteral verabreichbarer Lösungen aus stark sauren resp. stark basischen Salzen pharmazeutischer Wirkstoffe, dadurch gekennzeichnet, dass man ein hinreichend lösliches Salz eines solchen Wirkstoffes mit einem ausreichenden Volumen eines neutralisierenden Solvens zusammenbringt und gegebenenfalls entstehende und vorübergehend ausfallende Wirkstoff-Base resp. Wirkstoff-Säure durch leichtes Schütteln in Lösung bringt.

2. Verfahren nach Anspruch 1, dadurch gekennzeichnet, dass die pharmazeutischen Wirkstoffe sehr schwache Basen (pKs < 4) resp. sehr schwache Säuren (pKs > 8-9) darstellen.

3. Verfahren nach den Ansprüchen 1-2, dadurch gekennzeichnet, dass der Wirkstoff 5-Fluorcytosin ist.

4. Verfahren nach den Ansprüchen 1-3, dadurch gekennzeichnet, dass das Wirkstoffsalz 5-Fluorcytosin-Monohydrochlorid ist.

5. Verfahren nach den Anprüchen 1-4, dadurch gekennzeichnet, dass das Solvens zur Neutralisation des stark sauren resp. stark basischen Salzes eine oder mehrere, pharmazeutisch anwendbare Base(n) resp. Säure(n) enthält.

6. Verfahren nach Anspruch 5, dadurch gekennzeichnet, dass die pharmazeutisch anwendbare Base Dinatriumhydrogenphosphat, L-Arginin oder Tris(hydroxymethyl)aminomethan ist.

7. Verfahren nach Anspruch 5, dadurch gekennzeichnet, dass die pharmazeutische anwendbare Säure Citronensäure, Essigsäure oder Natriumdihydrogenphosphat ist.

8. Verfahren nach den Ansprüchen 1-7, dadurch gekennzeichnet, dass weitere, pharmazeutisch anwendbare Hilfsstoffe zur Isoosmotisierung, pH-Einstellung, Pufferung und Stabilisierung des Wirkstoffes, gemischt mit dem Wirkstoffsalz und/oder gelöst im Solvens, verwendet werden.

9. Verfahren nach den Ansprüchen 1-8, dadurch gekennzeichnet, dass Natriumhydrogencarbonat als Hilfsstoff verwendet wird.

10. Kit zur Herstellung von parenteral verabreichbaren Lösungen, umfassend:

a) einen Behälter, in dem sich ein stark saures resp. stark basisches Salz eines pharmazeutischen Wirkstoffes befindet, das hinreichend löslich und im trockenen Zustand stabil ist;

b) einen Behälter, in dem sich ein ausreichendes Volumen eines neutralisierenden Solvens befindet, das die vollständig bzw. nur teilweise entstehende Wirkstoff-Base resp. Wirkstoff-Säure auflösen kann; und

c) ein Verbindungsstück, das für die Ueberführung des Wirkstoffsalzes in das neutralisierende Solvens geeignet ist.

11. 5-Fluorcytosin-Monohydrochlorid.

12. 5-Fluorcytosin-Monohydrochlorid zur Verwendung bei der Herstellung von parenteralen Lösungen.